# EUROPEAN PATENT SPECIFICATION

(11) **EP 3 246 058 B1**
(45) Date of publication and mention of the grant of the patent: **13.03.2019**
(21) Application number: 17171230.0
(22) Date of filing: 16.05.2017
(51) Int. Cl.: A61M 5/162, A61M 39/10, A61M 39/26, A61M 39/00

(54) **DEVICE FOR THE PREPARATION AND ADMINISTRATION OF LIQUID SUBSTANCES, PARTICULARLY ANTIBLASTIC CHEMOTHERAPY DRUGS**
VORRICHTUNG ZUR HERSTELLUNG UND VERABREICHUNG VON FLÜSSIGEN SUBSTANZEN, INSBESONDERE VON ANTIBLASTISCHEN CHEMOTHERAPIEARZNEIMITTELN
DISPOSITIF DE PRÉPARATION ET D'ADMINISTRATION DE SUBSTANCES LIQUIDES, NOTAMMENT DE MÉDICAMENTS DE CHIMIOTHÉRAPIE ANTIBLASTIQUES

(30) Priority: 16.05.2016 IT UA20167179 U
(43) Date of publication of application: 22.11.2017
(73) Proprietor: BTC Medical Europe S.R.L., 37067 Valeggio sul Mincio (VR) (IT)
(72) Inventor: MARCHI, Mauro, 37067 Valeggio sul Mincio (VR) (IT)
(74) Representative: Zoli, Filippo

(56) References cited:
- US-A- 4 340 049
- US-A1- 2012 330 246

## Description

The present invention as defined in the claims relates to a device for the preparation and administration of liquid substances, particularly antiblastic chemotherapy drugs.

In the biomedical sector of the medical devices for the preparation and administration of drugs, in particular antiblastic chemotherapy drugs, the need is increasingly felt for optimizing and making more secure the preparation operations of the drugs to be administered to patients with oncologic diseases.

The proper implementation of these operations is therefore of paramount importance both for healthcare staff, as they have to handle particularly toxic substances, and for patients who are given the drugs.

The preparation devices of known type generally comprise a first connection element to a first container for feeding a first liquid substance, a second connection element connectable to an infusion line of a patient and a joining element interposed between the first and the second connection element and provided with a feeding channel of at least a second liquid substance. The joining element defines a passage channel of the liquid substances that connects the first connection element to the second connection element in a fluid-operated manner.

More particularly, through the first connection element is fed a pre-diluted solution required for the preparation of the patient for the administration of the second liquid substance, of the type of antiblastic chemotherapy drugs.

Generally, the preparation solution is initially administered to the patient, after which the chemotherapy drug is fed through the joining element.

The joining elements of known type can comprise one or more feeding ducts, arranged substantially in a Y with respect to the longitudinal axis of the joining element, of second liquid substances which are different to one another.

Along each duct is arranged at least one valve element that prevents reflux due to parallel infusion of liquid substances in the various branches.

Furthermore, the devices of known type are provided with blocking means for blocking the flow of the liquid substances which are manually removable by healthcare professionals depending on whether the patient requires the infusion or not.

In the present case, such blocking means obstruct the passage channel in order to avoid accidental leakage of the liquid substances prior to the connection of the second connection element to the infusion system.

These devices of known type do have some drawbacks.

The main drawback is related to the accidental spreading of the liquid substances, and particularly of the second liquid substance of the type of an antiblastic chemotherapy drug, prior to the administration of the liquid substances to the patient.

The blocking means of known type are, in fact, manually releasable and, in the case of accidental shocks or sudden maneuvers by the healthcare professionals, the risk of spreading the liquid substances is particularly high.

Another drawback is related to the waste of antiblastic chemotherapy drugs, in the event of accidental spread, and to the related costs of supplying in charge of the healthcare system.

Another drawback is related to the high risk of contamination of the healthcare professionals who are responsible for the preparation and administration of the antiblastic chemotherapy drugs in the event of accidental spreading of the drugs themselves.

Others devices are known from US 2012/330246 and US 4,340,049.

US2012/0330246 discloses a device for the preparation and administration of the liquid substances comprising a spike receiving subsystem which is manually interposed between a spike of an IV set and a needle-less syringe connector affixed to a hazardous drug container to provide a closed pathway for delivering hazardous drugs.

US 4,340,049 disclose a frangible or breakaway valve for use in a flexible tube. The valve comprises two parts: the tubular portion with a closed end an elongated handle breakable attached to the closed end. The valve is opened by breaking away the elongated, generally rigid handle and moving "walking" the rigid handle down the tube by folding the tube back and ford upon itself.

The main aim of the present invention is to provide a device for the preparation and administration of the liquid substances, particularly antiblastic chemotherapy drugs, which prevents the accidental spreading of the antiblastic chemotherapy drugs as a result of accidental maneuvers during their relevant handling by the healthcare staff.

Within this aim, one object of the present invention is to provide a device for the preparation and administration of liquid substances, particularly antiblastic chemotherapy drugs, which allows reducing the wastes of the drugs themselves, during the preparation and/or administration operations, and the related supply costs.

One object of the present invention is to provide a device which allows reducing the contamination of the healthcare professionals which are responsible for the preparation and administration of the drugs themselves, which have a high degree of toxicity.

Another object of the present invention is to provide a device for the preparation and administration of liquid substances, particularly antiblastic chemotherapy drugs, which allows to overcome the mentioned drawbacks of the prior art within the ambit of a simple, rational, easy, effective to use and low cost solution.

The above mentioned objects are achieved by the present device for the preparation and administration of liquid substances, particularly antiblastic chemotherapy drugs, having the characteristics of claim 1.

Other characteristics and advantages of the present invention will become more evident from the description of a preferred, but not exclusive, embodiment of a device for the preparation and administration of liquid substances, particularly antiblastic chemotherapy drugs, illustrated by way of an indicative, but nonlimiting example in the accompanying drawings, wherein:
Figure 1 is a front view of the device according to the invention in a first embodiment;
Figure 2 is a front view of the device of Figure 1 in a configuration of use;
Figure 3 is a front view of a detail of the device of Figure 1 in a closure configuration;
Figure 4 is a front view of a detail of the device of Figure 1 in an opening configuration;
Figure 5 is a front view of the device according to the invention in a second embodiment.

With particular reference to these illustrations, globally indicated with reference numeral 1 is a device for the preparation and administration of liquid substances, particularly antiblastic chemotherapy drugs.

The device 1 comprises a first connection element 2 connectable in a fluid-operated manner to a first container 3, e.g. of the type of intravenous drips, bags, ecoflac and the like, containing a first liquid substance A.

Generally, the first liquid substance A is of the type of a pre-diluted solution which is preparative for the administration of one or more liquid substances containing the specific drugs for the patients' therapy.

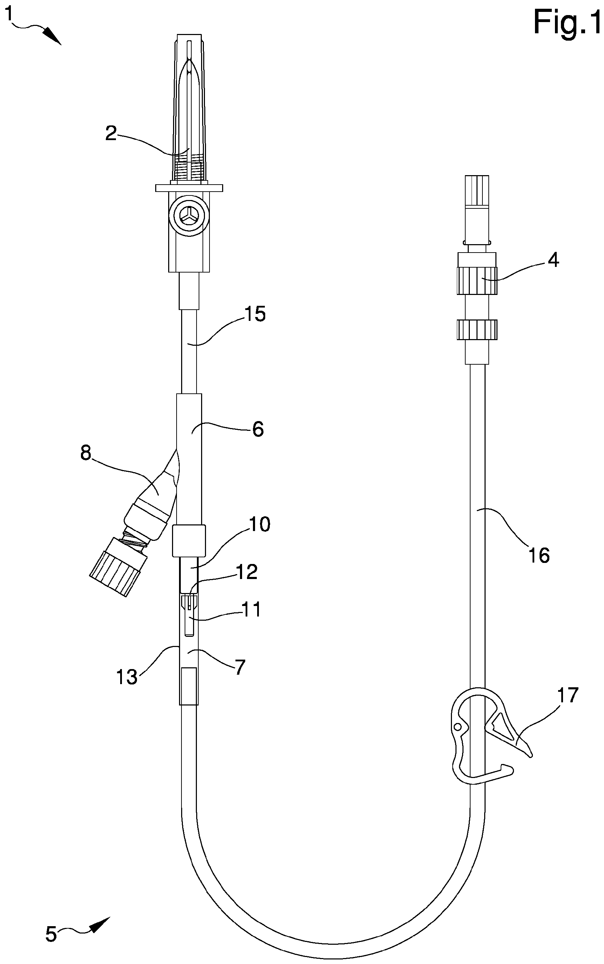

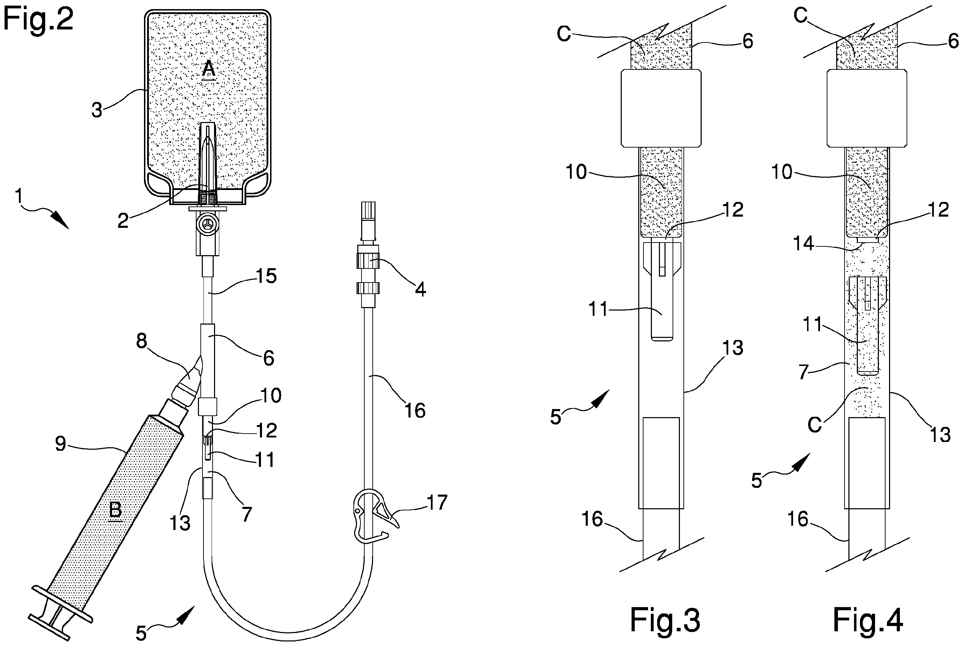

In a first embodiment shown in Figures 1 to 4, the first connection element 2 is of the type of a perforator with at least one way comprising a spike insertable inside the first container 3 and is provided with an air intake element provided with an antibacterial filter, usable for intravenous drips, ecoflac or other containers of the first liquid substance A.

Advantageously, the air intake element is positioned at the spike and allows avoiding any drips and aerosols, for the correct execution of the infusion operations performed by the healthcare professional, and the correct conveying of the first liquid substance A inside the first connection element 2.

The first connection element 2 can comprise a capsule element adapted to the passage of a gaseous fluid for sterilizing and maintaining the sterility of the first connection element itself.

The device 1 comprises a second connection element 4 connectable to an infusion line of a patient.

The second connection element 4 is of the type of a luer lock male fitting connectable to the infusion line and provided with assembly means adapted to avoid accidental disconnections between the second connection element 4 and the infusion line itself.

The second connection element 4 is made of polymeric material, e.g. in thermoplastic material of the acrylonitrile-butadiene-styrene type.

Advantageously, the second connection element 4 is provided with a casing element equipped with an accidental anti-spreading mechanism which allows avoiding the spreading of the liquid substances to be administered prior to the connection operations of the second connection element 4 to the infusion line.

Moreover, the device 1 comprises joining means 5 which define a passage channel 7, which is adapted to connect the first connection element 2 to the second connection element 4 in a fluid-operated manner.

The joining means 5 further comprise a feeding channel 8 of a second liquid substance B communicating with the passage channel 7 to obtain a mixture C.

The feeding channel 8 is connectable to a second container 9, generally of the syringe type, containing the second liquid substance B.

The feeding channel 8 has a connection of the luer lock female type which is adapted to connect the second container 9 to the feeding channel itself in a fluid-operated manner.

Generally, the second liquid substance B is substantially of the type of antiblastic chemotherapy drugs.

Within the scope of the present discussion, with the term mixture is meant the liquid substance obtained by mixing the liquid substances A, B and which takes place at the junction between the passage channel 7 and the feeding channel 8.

The flow of the mixture C propagates along the passage channel 7 up to the second connection element 4.

Usefully, the joining means 5 comprise a joining element 6 which is interposed between the first connection element 2 and the second connection element 4 and is made of a transparent plastic material, e.g. of the polycarbonate (PC) type.

More in detail, the joining element 6 has a substantially Y-shaped conformation provided with a main branch, which defines at least one portion of the passage channel 7, and at least a secondary branch, defining the feeding channel 8, arranged transversely with respect to the main branch.

Preferably, the joining element 6 comprises at least one self-sealing and bidirectional regulator element arranged along the feeding channel 8 and made, e.g., of silicone material.

Conveniently, the feeding channel 8 comprises a capsule closure element adapted to preserve the sterility of the feeding channel itself until the device 1 is used for the first time.

At the same time when the second container 9 is connected to the feeding channel 8, an operation being carried out by the healthcare professional, the regulator element opens automatically so that the flow of the second liquid substance B flows freely and without friction along the feeding channel 8.

According to the invention, the joining means 5 comprise removable security means 10, 11, 12 which are interposed between the feeding channel 8 and the second connection element 4 and are adapted to temporarily obstruct the passage channel 7 to avoid the accidental spreading of the mixture C prior to the connection of the second connection element 4 to the infusion line.

Preferably, the security means 10, 11, 12 are of the breaking type and are removable by means of the manual and voluntary action of the healthcare professional.

The breaking of the security means 10, 11, 12 causes the mixture C to flow along the section of the passage channel 7 defined between the security means themselves and the second connection element 4.

More specifically, the security means 10, 11, 12 comprise a first breakable portion 10, locked together with the joining element 6 and defining one part of the passage channel 7, and a second breakable portion 11 associated with the first portion 10, along a predefined breaking line 12 to define a single body piece, and separable from it to allow the passage of the mixture C towards the second connection element 4.

Suitably, the first portion 10 and the second portion 11 are made of a rigid material.

More specifically, the joining means 5 comprise a connecting length 13 made of a flexible material and the security means 10, 11, 12 are arranged inside the connecting length 13.

As can be seen from the illustrations, the connecting length 13 is interposed between the joining element 6 and the second connection element 4.

More particularly, the joining element 6 and the connecting length 13 are two separate bodies and hermetically associated so as to be connected to each other in a fluid-operated manner to define an extension of the passage channel 7 between the joining element 6 and the second connection element 4.

The security means 10, 11, 12 are contained inside the connecting length 13 so as to temporarily obstruct the passage channel 7 to prevent the flow of the mixture C from passing along the passage channel itself.

Alternative embodiments cannot however be ruled out wherein the connecting length 13 and the joining element 6 are made in a single body piece.

The security means 10, 11, 12 have:
- a closure configuration, in which the first portion 10 and the second portion 11 are associated with each other along the predefined breaking line 12 for blocking the passage channel 7; and
- an opening configuration, in which the second portion 11 is separated from the first portion 10 so as to define a passage opening 14 of the mixture C towards the second connection element 4.

The switching between the closure configuration and the opening configuration is performed by means of the application by the healthcare staff of forces, e.g. opposing each other, onto the portions of the connecting length 13 arranged at the first portion 10 and the second portion 11, so as to dissociate them at the predefined breaking line 12.

More specifically, as a result of the switching between the closure configuration and the opening configuration, the first portion 10 is associated with the joining element 6, while the second portion 11 remains located inside the connecting length 13 and moved away from the first portion 10.

In the first embodiment shown in Figures 1 to 4, the joining means 5 comprise a first tubular element 15 interposed between the first connection element 2 and the joining element 6 and defining at least one part of the passage channel 7 for the passage of the first liquid substance A.

In this first embodiment, the joining means 5 also comprise a second tubular element 16 interposed between the connecting length 13 and the second connection element 4 and defining at least one part of the passage channel 7 for the passage of the mixture C.

Alternative embodiments cannot be ruled out wherein the first tubular element 15, the joining element 6, the connecting length 13 and the second tubular element 16 are made in a single body piece defining the joining means 5.

Usefully, the first tubular element 15 and the second tubular element 16 are made of a polymeric material which is pigmented for the filtering of light beams having a wavelength substantially comprised between 200 nm and 600 nm.

The mixture C, and therefore the antiblastic chemotherapy drug, is sensitive to light radiation having a wavelength comprised between 200 nm and 600 nm.

Therefore, the particular pigmentation of the tubular elements 15, 16 is such as to maintain the transparency of the tubular elements themselves and allow the filtration of the aforementioned radiation, which is mainly responsible for the inactivation of the photosensitive substances present in the mixture C, while maintaining the properties of the mixture C as much as possible unchanged from preparation to administration to the patient.

More specifically, the tubular elements 15, 16 are made of amber polyvinyl chloride having a phthalate-free medical grade (DEHP FREE).

Alternative embodiments cannot however be ruled out wherein the tubular elements 15, 16 are made of alternative materials suitable to be used in the medical field.

Advantageously, the device 1 comprises removable clamping means 17 which are associated with the second tubular element 16 for the blocking of the flow of the mixture C along the passage channel 7.

The clamping means 17 are substantially of the type of a clamp and are movable between an open configuration, in which they allow for the passage of the mixture C along the passage channel 7 inside the second tubular element 16, and a closed configuration, in which they block the passage of the mixture C along the passage channel 7.

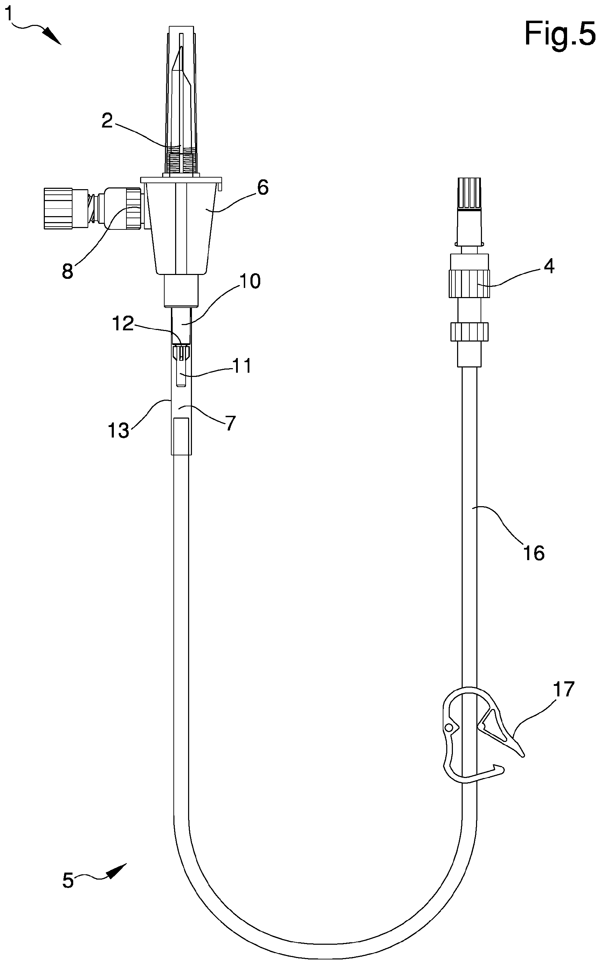

In a second embodiment shown in Figure 5, the first connection element 2 and the joining means 5 are made in a single body piece.

More specifically, the first connection element 2 is provided with the spike only that can be inserted inside the first container 3, and is free of the air intake element described previously.

The first connection element 2 thus obtained is mostly used when the container 3 is of the type of a bag and the infusion operations of the first liquid substance A from the container 3 to the first connection element itself are free of drips and aerosols.

As can be seen from Figure 5, the joining element 6 is made integral to the first connection element 2.

In the same way as the first embodiment, the feeding channel 8 is arranged transversely to the passage channel 7 and the connecting length 13 is associated with the joining element 6.

In the second embodiment shown in Figure 5, the joining means 5 are free of the first tubular element 15 since the joining element 6 is locked together with the first connection element 2.

At the same time, the joining means 5 comprise the second tubular element 16 interposed between the connecting length 13 and the second connection element 4 and defining an extension of the passage channel 7.

In the same way as the first embodiment, also in the second embodiment, the device 1 can comprise removable clamping means 17 which are associated with the second tubular element 16 to block the flow of the mixture C along the passage channel 7.

It has in practice been found that the disclosed invention achieves the intended objects and in particular it is emphasized that the device thus obtained allows avoiding the accidental spreading of antiblastic chemotherapy drugs as a result of unexpected or unwanted maneuvers during the handling of the device itself by the healthcare staff.

The manually removable security means, which are arranged at the junction point between the passage channel and the feeding channel in which the liquid substances mix together and flow in turn simultaneously along the passage channel, ensure the block of the mixture until the activation thereof by the healthcare staff as a result of the connection of the device to the administration means.

It follows, therefore, that once the container of the antiblastic chemotherapy drugs is connected to the feeding channel, until the healthcare staff breaks the security means, the flow of the mixture composed of the first and of the second liquid substance is obstructed by the security means themselves.

In particular, the security means avoid any accidental spreading of the antiblastic chemotherapy drugs during their preparation and prior to their administration, thus preserving the environments and health of people who may be contaminated by such highly toxic substances.

## Claims

1. Device (1) for the preparation and administration of liquid substances, particularly antiblastic chemotherapy drugs, comprising:
- at least a first connection element (2) connectable to at least a first container (3) containing a first liquid substance (A);
- at least a second connection element (4) connectable to at least one infusion line of a patient;
- joining means (5) which define at least one passage channel (7), which is adapted to connect said first connection element (2) to said second connection element (4) in a fluid-operated manner and comprising at least one feeding channel (8) of at least a second liquid substance (B) communicating with said passage channel (7) to obtain at least one mixture (C);
**characterized by** the fact that said joining means (5) comprise removable security means (10, 11, 12) which are interposed between said feeding channel (8) and said second connection element (4) and adapted to temporarily obstruct said passage channel (7).

2. Device (1) according to claim 1, **characterized by** the fact that said security means (10, 11, 12) are of the breaking type.

3. Device (1) according to one or more of the preceding claims, **characterized by** the fact that said security means (10, 11, 12) comprise at least a first portion (10), locked together with said joining means (5) and defining one part of said passage channel (7), and at least a second portion (11) associated with said first portion (10) along at least one predefined breaking line (12) to define a single body piece and separable from it to allow the passage of the mixture (C) towards said second connection element (4).

4. Device (1) according to one or more of the preceding claims, **characterized by** the fact that said security means (10, 11, 12) have:
- a closure configuration, in which said first portion (10) and said second portion (11) are associated with each other along said predefined breaking line (12) for the obstruction of said passage channel (7);
- and an opening configuration, in which said second portion (11) is separated from said first portion (10), the latter defining a passage opening (14) of said mixture (C) towards said second connection element (4).

5. Device (1) according to one or more of the preceding claims, **characterized by** the fact that said joining means (5) comprise at least a connecting length (13) made of a flexible material and by the fact that said security means (10, 11, 12) are arranged inside said connecting length (13).

6. Device (1) according to one or more of the preceding claims, **characterized by** the fact that said joining means (5) comprise at least a first tubular element (15) interposed between said first connection element (2) and said feeding channel (8) for the passage of said first liquid substance (A).

7. Device (1) according to one or more of the preceding claims, **characterized by** the fact that said joining means (5) comprise at least a second tubular element (16) interposed between said feeding channel (8) and said second connection element (4) for the passage of said mixture (C).

8. Device (1) according to one or more of the preceding claims, **characterized by** the fact that said first tubular element (15) and said second tubular element (16) are made of a polymeric material which is pigmented for the filtering of light beams having a wavelength substantially comprised between 200 nm and 600 nm.

9. Device (1) according to one or more of the preceding claims, **characterized by** the fact that said first connection element (2) and said joining means (5) are made in a single body piece.

10. Device (1) according to one or more of the preceding claims, **characterized by** the fact that it comprises removable clamping means (17) which are associated with said second tubular element (16), said clamping means (17) being movable between an open configuration, in which they allow for the passage of said mixture (C) along said passage channel (7), and a closed configuration, in which they block the passage of the flow of said mixture (C) along said passage channel (7).

## Patentansprüche

1. Vorrichtung (1) für die Vorbereitung und Verabreichung von flüssigen Substanzen, insbesondere antiblastischen Chemotherapie-Medikamenten, umfassend:
- mindestens ein erstes Verbindungselement (2), das verbindbar ist mit mindestens einem ersten Behälter (3), der eine erste flüssige Substanz (A) enthält;
- mindestens ein zweites Verbindungselement (4), das verbindbar ist mit mindestens einer Infusionsleitung eines Patienten;
- Verbindungsmittel (5), die mindestens einen Durchgangskanal (7) definieren, welcher dazu ausgelegt ist, das erste Verbindungselement (2) mit dem zweiten Verbindungselement (4) in einer fluid-betriebenen Weise zu verbinden und der mindestens einen Zuführungskanal (8) mindestens einer zweiten flüssigen Substanz (B) umfasst, die mit dem Durchgangskanal (7) in Verbindung steht, um mindestens eine Mischung (C) zu erhalten;
**dadurch gekennzeichnet, dass** die Verbindungsmittel (5) lösbare Sicherheitsmittel (10, 11, 12) umfassen, die zwischen dem Zuführungskanal (8) und dem zweiten Verbindungselement (4) eingefügt sind und dazu ausgelegt sind, temporär den Durchgangskanal (7) zu blockieren.

2. Vorrichtung (1) nach Anspruch 1, **dadurch gekennzeichnet, dass** die Sicherheitsmittel (10, 11, 12) von dem Bruchtyp sind.

3. Vorrichtung (1) nach einem oder mehreren der vorhergehenden Ansprüche, **dadurch gekennzeichnet, dass** die Sicherheitsmittel (10, 11, 12) mindestens einen ersten Teil (10), der zusammengeschlossen ist mit den Verbindungsmitteln (5) und der einen Bestandteil des Durchgangskanals (7) definiert, und mindestens einen zweiten Teil (11) umfassen, der mit dem ersten Teil (10) entlang mindestens einer vordefinierten Bruchkante (12) verbunden ist, um ein einzelnes Körperstück zu definieren und der trennbar davon ist, um den Durchfluss der Mischung (C) zu dem zweiten Verbindungselement (4) zu erlauben.

4. Vorrichtung (1) nach einem oder mehreren der vorhergehenden Ansprüche, **dadurch gekennzeichnet, dass** die Sicherheitsmittel (10, 11, 12) aufweisen:
- eine Verschlusskonfiguration, in der der erste Teil (10) und der zweite Teil (11) miteinander entlang der vordefinierten Bruchkante (12) zur Blockierung des Durchgangskanals (7) verbunden sind;
- und eine Öffnungskonfiguration, in der der zweite Teil (11) von dem ersten Teil (10) getrennt ist, wobei letzterer eine Durchgangsöffnung (14) für die Mischung (C) zu dem zweiten Verbindungselement (4) definiert.

5. Vorrichtung (1) nach einem oder mehreren der vorhergehenden Ansprüche, **dadurch gekennzeichnet, dass** die Verbindungsmittel (5) mindestens eine Verbindungslänge (13) umfassen, die aus einem flexiblen Material gemacht ist und dadurch, dass die Sicherheitsmittel (10, 11, 12) innerhalb der Verbindungslänge (13) angeordnet sind.

6. Vorrichtung (1) nach einem oder mehreren der vorhergehenden Ansprüche, **dadurch gekennzeichnet, dass** die Verbindungsmittel (5) mindestens ein erstes röhrenförmiges Element (15) umfassen, das zwischen dem ersten Verbindungselement (2) und dem Zuführungskanal (8) zum Durchfluss der ersten flüssigen Substanz (A) eingefügt ist.

7. Vorrichtung (1) nach einem oder mehren der vorhergehenden Ansprüche, **dadurch gekennzeichnet, dass** die Verbindungsmittel (5) mindestens ein zweites, röhrenförmiges Element (16) umfassen, das zwischen dem Zuführungskanal (8) und dem zweiten Verbindungselement (4) zum Durchfluss der Mischung (C) eingefügt ist.

8. Vorrichtung (1) nach einem oder mehreren der vorhergehenden Ansprüche, **dadurch gekennzeichnet, dass** das erste, röhrenförmige Element (15) und das zweite, röhrenförmige Element (16) aus einem Polymermaterial hergestellt sind, das pigmentiert ist zur Filterung von Lichtstrahlen, die eine Wellenlänge im Wesentlichen umfasst zwischen 200 nm und 600 nm aufweisen.

9. Vorrichtung (1) nach einem oder mehreren der vorhergehenden Ansprüche, **dadurch gekennzeichnet, dass** das erste Verbindungselement (2) und die Verbindungsmittel (5) in einem einzelnen Körperstück hergestellt sind.

10. Vorrichtung (1) nach einem oder mehreren der vorhergehenden Ansprüche, **dadurch gekennzeichnet, dass** es lösbare Klemmmittel (17) umfasst, die mit dem zweiten, röhrenförmigen Element (16) verbunden sind, wobei die Klemmmittel (17) beweglich sind zwischen einer offenen Konfiguration, in der sie den Durchfluss der Mischung (C) entlang des Durchgangskanals (7) erlauben, und einer geschlossenen Konfiguration, in der sie den Durchfluss der Mischung (C) entlang des Durchgangskanals (7) blockieren.

## Revendications

1. Dispositif (1) de préparation et d'administration de substances liquides, en particulier de médicaments de chimiothérapie antiblastiques, comprenant :
- au moins un premier élément de raccordement (2) pouvant être raccordé à au moins un premier contenant (3) contenant une première substance liquide (A) ;
- au moins un second élément de raccordement (4) pouvant être raccordé à au moins une ligne de perfusion d'un patient ;
- des moyens de jonction (5) qui définissent au moins un canal de passage (7), qui est adapté pour raccorder ledit premier élément de raccordement (2) audit second élément de raccordement (4) d'une manière actionnée par un fluide et comprenant au moins un canal d'alimentation (8) d'au moins une seconde substance liquide (B) communiquant avec ledit canal de passage (7) pour obtenir au moins un mélange (C) ;
**caractérisé par le fait que** lesdits moyens de jonction (5) comprennent des moyens de sécurité amovibles (10, 11, 12) qui sont interposés entre ledit canal d'alimentation (8) et ledit second élément de raccordement (4) et adaptés pour obstruer temporairement ledit canal de passage (7).

2. Dispositif (1) selon la revendication 1, **caractérisé par le fait que** lesdits moyens de sécurité (10, 11, 12) sont du type à rupture.

3. Dispositif (1) selon une ou plusieurs des revendications précédentes, **caractérisé par le fait que** lesdits moyens de sécurité (10, 11, 12) comprennent au moins une première portion (10), verrouillée conjointement avec lesdits moyens de jonction (5) et définissant une partie dudit canal de passage (7), et au moins une seconde portion (11) associée à ladite première portion (10) le long d'au moins une ligne de rupture prédéfinie (12) pour définir une pièce en un seul corps et séparable de celle-ci pour permettre le passage du mélange (C) vers ledit second élément de raccordement (4).

4. Dispositif (1) selon une ou plusieurs des revendications précédentes, **caractérisé par le fait que** lesdits moyens de sécurité (10, 11, 12) comportent :
- une configuration de fermeture, dans laquelle ladite première portion (10) et ladite seconde portion (11) sont associées l'une à l'autre le long de ladite ligne de rupture prédéfinie (12) pour l'obstruction dudit canal de passage (7) ;
- et une configuration d'ouverture, dans laquelle ladite seconde portion (11) est séparée de ladite première portion (10), cette dernière définissant une ouverture de passage (14) dudit mélange (C) vers ledit second élément de raccordement (4).

5. Dispositif (1) selon une ou plusieurs des revendications précédentes, **caractérisé par le fait que** lesdits moyens de jonction (5) comprennent au moins une longueur de raccordement (13) fabriquée en un matériau souple et **par le fait que** lesdits moyens de sécurité (10, 11, 12) sont agencés à l'intérieur de ladite longueur de raccordement (13).

6. Dispositif (1) selon une ou plusieurs des revendications précédentes, **caractérisé par le fait que** lesdits moyens de jonction (5) comprennent au moins un premier élément tubulaire (15) interposé entre ledit premier élément de raccordement (2) et ledit canal d'alimentation (8) pour le passage de ladite première substance liquide (A).

7. Dispositif (1) selon une ou plusieurs des revendications précédentes, **caractérisé par le fait que** lesdits moyens de jonction (5) comprennent au moins un second élément tubulaire (16) interposé entre ledit canal d'alimentation (8) et ledit second élément de raccordement (4) pour le passage dudit mélange (C).

8. Dispositif (1) selon une ou plusieurs des revendications précédentes, **caractérisé par le fait que** ledit premier élément tubulaire (15) et ledit second élément tubulaire (16) sont fabriqués en un matériau polymère qui est pigmenté pour la filtration des faisceaux lumineux ayant une longueur d'onde sensiblement comprise entre 200 nm et 600 nm.

9. Dispositif (1) selon une ou plusieurs des revendications précédentes, **caractérisé par le fait que** ledit premier élément de raccordement (2) et lesdits moyens de jonction (5) sont fabriqués en une pièce en un seul corps.

10. Dispositif (1) selon une ou plusieurs des revendications précédentes, **caractérisé par le fait qu'**il comprend des moyens de serrage amovibles (17) qui sont associés audit second élément tubulaire (16), lesdits moyens de serrage (17) étant mobiles entre une configuration ouverte, dans laquelle ils permettent le passage dudit mélange (C) le long dudit canal de passage (7), et une configuration fermée, dans laquelle ils bloquent le passage du flux dudit mélange (C) le long dudit canal de passage (7).
